# EUROPEAN PATENT SPECIFICATION

(11) **EP 3 459 514 B1**
(45) Date of publication and mention of the grant of the patent: **04.12.2019**
(21) Application number: 17823898.6
(22) Date of filing: 26.05.2017
(51) Int. Cl.: A61F 13/53, A61F 13/535, A61F 13/536, A61F 13/537, A61F 13/513, A61F 13/532, A61F 13/84, A61F 13/533, A61F 13/539

(54) **ABSORBENT ARTICLE**
SAUGFÄHIGER ARTIKEL
ARTICLE ABSORBANT

(30) Priority: 07.07.2016 JP 2016135114
(43) Date of publication of application: 27.03.2019
(73) Proprietor: Unicharm Corporation, Ehime 799-0111 (JP)
(72) Inventor: YAMAGUCHI, Masashi, Kanonji-shi Kagawa 769-1602 (JP); GODA, Hiroki, Kanonji-shi Kagawa 769-1602 (JP)
(74) Representative: Dolleymores
(86) International application number: PCT/JP2017/019785
(87) International publication number: WO 2018/008291

(56) References cited:
- EP-A2- 1 252 873
- EP-A2- 2 919 737
- JP-A- 2004 166 832
- JP-A- 2008 264 084
- JP-A- 2011 212 211
- JP-A- 2014 068 681
- US-A1- 2003 135 174
- US-A1- 2007 087 169
- US-A1- 2015 080 837

## Description

### FIELD

The present invention relates to an absorbent article such as a disposable diaper, incontinence pad or sanitary napkin.

### BACKGROUND

Absorbent articles have been disclosed that have recesses in the absorbent body formed by embossing, the recesses being colored with a different color than the regions other than the recesses (PTL 1). In PTL 1, a colored layer is placed in contact with the back side of a liquid-permeable sheet where recesses are to be provided, and the recesses are formed by embossing.

Another absorbent article that has been disclosed has permanent channels that do not include super-absorbent polymer particles in the absorbent core of the absorbent article, while also having a printed adhesive layer that indicates channels, between the top sheet and the absorbent core (PTL 2). PTL 2 teaches that the absorbent article may also include embossing to indicate the channels.

There has also been disclosed a method in which, when components of an absorbent article are joined together with an adhesive, the sites bonded by heat or pressure for the joining process are colored (PTL 3).

### [CITATION LIST]

### [PATENT LITERATURE]

[PTL 1] Japanese Unexamined Patent Publication No. 2009-207684
[PTL 2] Japanese Unexamined Patent Publication No. 2015-533623
[PTL 3] Japanese Patent Publication No. 4787991

### SUMMARY

### [TECHNICAL PROBLEM]

In PTL 1, since the colored layer situated in contact with the back side of the liquid-permeable sheet is a hot-melt coloring agent, the colored layer is present up to the regions surrounding the design pattern formed by the recesses, but the color of the recesses and the color of the colored regions surrounding them are the same color, and no particular processing is carried out in the non-colored regions surrounding the colored regions in order to increase the visibility of the colored regions.

In PTL 2, embossing is also utilized in addition to the printed adhesive layer that serves to indicate the channels, and while the stamped grooves formed by embossing appear to be of a different color than the non-colored sections that are not the stamped grooves, the stamped grooves and the separate printed pattern together form a single pattern, with the coloration of the stamped grooves being coloration in an integral fashion with coloration of the rest of the pattern, and since such integral coloration has resulted in the same color in both the stamped grooves and the other regions, it has not been possible to display the stamped grooves in a more distinct manner. Also, in PTL 2, no particular processing is carried out for increased visibility in the non-colored regions surrounding the colored regions.

Even in PTL 3, no special consideration is given to increasing the visibility in the non-colored regions surrounding the colored regions.

Therefore, when the absorbent body absorbs body fluid and becomes stained with the color of the body fluid, the difference between the color of the original colored region and that of the surrounding region is reduced, and this has been problematic as it reduces the visibility of the original colored region, making it more difficult to be seen.

It is therefore an object of the present invention to provide an absorbent article having colored regions between the top sheet and the absorbent core, wherein the color produced by body fluid absorbed in the regions surrounding the colored regions is concealed to improve the visibility of the colored regions.

### [SOLUTION TO PROBLEM]

The present invention solves the problem described above and provides the following aspects.
(Aspect 1) An absorbent article having a liquid-permeable top sheet, a liquid-impermeable back sheet and an absorbent core situated between them, and having a thickness direction, wherein the absorbent article:
   has, between the top sheet and the absorbent core, a colored region that is visible through the top sheet and has a different color than the color of the top sheet,
   has, between the top sheet and the absorbent core, a liquid-permeable base sheet, the base sheet being constructed to include a fiber material, and
   has, in the fiber material of the base sheet, a microparticle-attaching region in which non-absorbent, water-insoluble microparticles having a different color than the color of the colored region have been attached, the average particle size of the microparticles being smaller than the average width of the fiber material of the base sheet, and
   the microparticle-attaching region includes a region that overlaps with both the colored region and the surrounding region surrounding the colored region, in the thickness direction.

   (Function and effect) Since the non-absorbent, water-insoluble microparticles (hereunder also referred to simply as "microparticles") having a color that differs from the color of the colored region are attached onto the liquid-permeable base sheet composed of a fiber material, situated between the top sheet and the absorbent core, the microparticles adhere to the fiber material surface of the base sheet but do not block the back side or the gaps between the fiber material and hence do not inhibit permeation of body fluids. Since non-absorbent, water-insoluble microparticles do not retain body fluid, the sections of the base sheet where the microparticles are attached do not retain body fluid, and they function to conceal the color of the base sheet and the absorbent body core further toward the non-skin side than the base sheet, that are stained by body fluid. Since the microparticles that can conceal the color produced by staining of the base sheet and absorbent core with body fluid are in a region overlapping in the thickness direction with the colored region and its surrounding region, the property of concealing the color of the colored region and its surrounding region is increased, and the colored region can be clearly seen even after absorption of body fluid. This results in less inhibition of the absorption property for body fluids, and allows the colored region to be clearly seen through the top sheet even after absorption of body fluids.
(Aspect 2) The absorbent article according to aspect 1, wherein the average particle size of the microparticles is in the range of 0.01 to 5 µm.
   (Function and effect) Since the average particle size of the non-water-absorbing, water-insoluble microparticles is in the range of 0.01 to 5 µm, the microparticles can cover the fiber material surface of the base sheet and ensure liquid permeability while improving the concealing property.
(Aspect 3) The absorbent article according to aspect 1 or 2, wherein the colored region overlaps with recesses and/or slits formed in the absorbent core, in the thickness direction of the absorbent article.
   (Function and effect) Since the colored region overlaps with recesses or slits formed in the absorbent core, the colored region can be more clearly seen by the level differences of the recesses or slits.
(Aspect 4) The absorbent article according to any one of aspects 1 to 3, wherein the microparticles are attached to the fiber material of the base sheet by a binder.
   (Function and effect) Since the non-water-absorbing, water-insoluble microparticles are attached to the fiber material of the base sheet by a binder, fewer of the microparticles migrate and fall off from the base sheet during production, during use and even after absorption of body fluid, and the concealing property is less likely to be reduced.
(Aspect 5) The absorbent article according to any one of aspects 1 to 4, wherein the colored region is in the base sheet.
   (Function and effect) By having the colored region and the microparticle-attaching region in the same sheet, the border between the colored region and the concealed section surrounding the colored region is sharper, the contrast is distinct, and the colored region can be more clearly seen.
(Aspect 6) The absorbent article according to any one of aspects 1 to 5, which further has a separate liquid-permeable base sheet different from the base sheet, further toward the top sheet side than the base sheet between the top sheet and the absorbent core, the colored region being in the separate base sheet.
   (Function and effect) Since the colored region is in a separate base sheet further toward the top sheet side than the base sheet with the concealing function, on which the non-water-absorbing, water-insoluble microparticles are attached, the colored region in the separate base sheet conceals coloration of the absorbent core by body fluid.
(Aspect 7) The absorbent article according to aspect 5, wherein the colored region is colored by a pressure sensitive coloring agent, and the microparticles are a developing agent for the pressure sensitive coloring agent.
   (Function and effect) Since a pressure sensitive coloring agent is used for coloration, allowing matching coloration to be obtained in the recesses formed by embossing, and the developing agent that expresses coloration of the pressure sensitive coloring agent also exhibits a concealing function as the non-water-absorbing, water-insoluble microparticles, contrast between the colored region and the concealed section surrounding the colored region is distinct and the embossing pattern and colored region can be more clearly seen.
(Aspect 8) The absorbent article according to any one of aspects 1 to 7, wherein the base sheet is tissue paper.
   (Function and effect) Since the tissue paper has higher fiber material density than the top sheet, an advantage is provided in that the density of the coloration before absorption of body fluid is also increased, and the colored region is easy to discern. With tissue paper, however, coloration of the colored region tends to be difficult to see due to body fluid retained after absorption of the body fluid, but if the non-water-absorbing, water-insoluble microparticles have been adsorbed, then liquid will be less likely to be retained and the colored pattern will be clearly seen.
(Aspect 9) The absorbent article according to any one of aspects 1 to 8, wherein the absorbent core is covered by a core wrap, the base sheet is the core wrap, and the colored region is in an embossed section formed in the core wrap and the absorbent core.
   (Function and effect) Since the colored region is in an embossed section formed in the absorbent body comprising an absorbent core covered by a core wrap, and the non-water-absorbing, water-insoluble microparticles are present in the colored region of the core wrap and its surrounding region, even when the absorbent body absorbs body fluid, the colored region of the core wrap is effectively concealed from the color produced by body fluid and the colored region coinciding with the embossed section can be distinctly seen, so that a hygienic feel and feeling of assurance are provided for the user.

### [ADVANTAGEOUS EFFECTS OF INVENTION]

According to the invention there is provided an absorbent article having a colored region between the top sheet and the absorbent core, wherein the color produced by body fluid absorbed in the colored region and its surrounding region is concealed to improve the visibility of the colored region.

### BRIEF DESCRIPTION OF DRAWINGS

FIG. 1 is an expanded plan view of a disposable diaper 1 as an embodiment of the present invention.
FIG. 2 is an end view of an end face along line II-II of FIG. 1.
FIG. 3 is a plan view of an absorbent body 10 included in a disposable diaper 1.
FIG. 4(A) is an electron micrograph of the surface of a core wrap of a comparative example, and FIG. 4(B) is an electron micrograph of the surface of a core wrap of a working example.
FIG. 5 is an end view of a disposable diaper according to another embodiment, similar to FIG. 2.
FIG. 6 is an end view of a disposable diaper according to yet another embodiment, similar to FIG. 2.
FIG. 7 is an end view of a disposable diaper according to yet another embodiment, similar to FIG. 2.
FIG. 8(A) is a perspective view of a section showing an example of a recess formed in an absorbent body according to a preferred embodiment, and Fig. 8(B) is a perspective view of a section showing the raised section of an embossing roll used to form the recess shown in Fig. 8(A).

### DESCRIPTION OF EMBODIMENTS

### (Embodiment of disposable diaper)

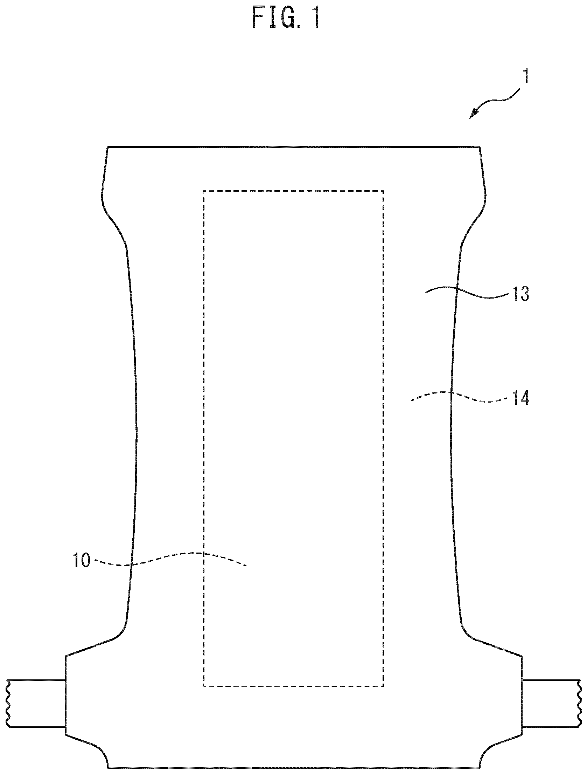

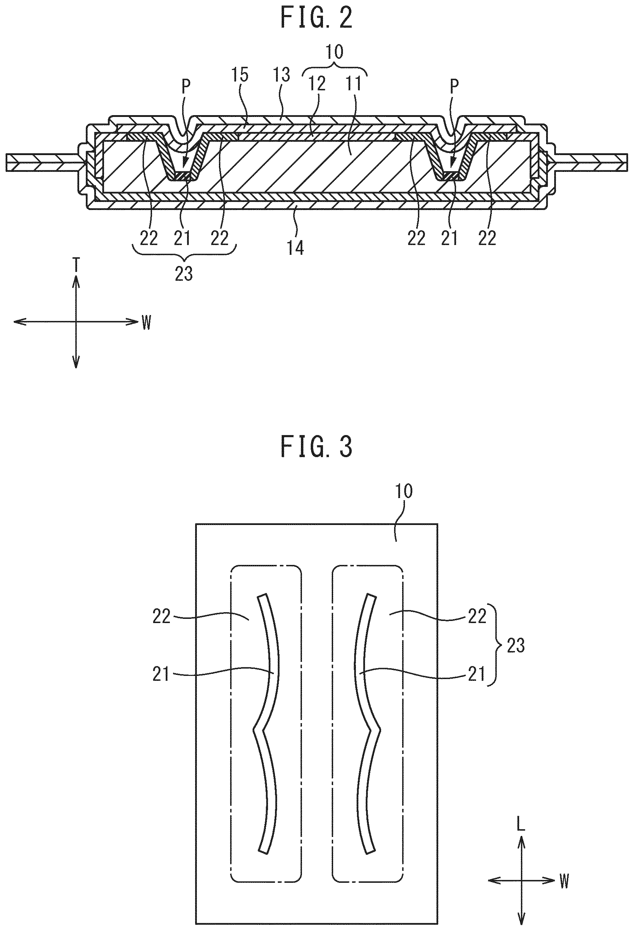

A disposable diaper 1 as an embodiment of the absorbent article of the invention will now be explained with reference to the accompanying drawings. Fig. 1 is an expanded plan view of the disposable diaper 1 as one embodiment, showing the disposable diaper 1 as seen from the skin side. Fig. 2 is an end view of an end face along line II-II of Fig. 1. Fig. 3 is a plan view of an absorbent body 10 included in a disposable diaper 1. As shown in Fig. 1 and Fig. 2, the disposable diaper 1 has a lengthwise direction L, a widthwise direction W, and a thickness direction T which is the direction perpendicular to the lengthwise direction L and widthwise direction W.

Referring to Fig. 1 and Fig. 2, the disposable diaper 1 has an absorbent body 10, a liquid-permeable top sheet 13 on the skin side of the absorbent body 10, and a liquid-impermeable back sheet 14 on the non-skin side of the absorbent body 10. For this embodiment, the disposable diaper 1 further has a liquid-permeable second sheet 15 between the absorbent body 10 and the top sheet 13, as a liquid-trapping layer. According to the invention, the second sheet 15 may optionally be omitted.

The top sheet 13 of the disposable diaper of this embodiment is optically transparent, so that the colored region formed in the absorbent body 10 is visible. The degree of optical transparency may be evaluated by the light transmittance, but in this case it is important for the colored region formed in the absorbent body 10 to be visible. For example, the light transmittance may be 60 to 95% and is preferably 65 to 90%, but since this will depend on the density of coloration of the colored region formed in the absorbent body 10, the light transmittance cannot be specified for all cases. Moreover, in addition to being optically transparent, the top sheet 13 may also have an opening, with the colored region formed in the absorbent body 10 being visible through the opening.

When a second sheet 15 is present and the colored region is further toward the non-skin side than the second sheet 15, as in this embodiment, the second sheet 15 must also be optically transparent so that the colored region will be visible. In this case, the total light transmittance of the top sheet 13 and the second sheet 15 may preferably be 50 to 90%, for example, and further 55 to 85%.

The absorbent body 10 includes an absorbent core 11 typically comprising ground pulp and an absorbent polymer (SAP), and a liquid-permeable core wrap 12 covering the absorbent core 11, and typically being made of tissue paper.

Referring to Fig. 3, the absorbent body 10 has recesses P which are a pair of left and right embossed sections on both the left and right sides in the widthwise direction W of the absorbent body 10, and which serve as curving origins (hinges) for the absorbent body, extending in the lengthwise direction. Referring to Fig. 2 and Fig. 3, the recesses P of the embossed sections are recesses (compressed recesses) running from the surface of the core wrap 12 to the interior of the absorbent core 11 in the thickness direction T. The present invention is preferably applied to the colored regions in the recesses (compressed recesses), the embossed recesses, running from the surface of the core wrap to the interior of the absorbent core in the thickness direction.

Referring to Fig. 2 and Fig. 3, in the recesses P of the embossed sections of the absorbent body 10, the core wrap 12 is colored, forming colored regions 21. The colored regions 21 are blue, for example. Since the colored regions 21 are formed in the recesses P formed by embossing, the recesses P and the colored regions 21 coincide. However, the recesses P and the colored regions 21 do not need to be completely matching, and the colored regions 21 may constitute parts of the recesses P, or they may be slightly wider than the recesses P.

The colored regions 21 of the core wrap 12 and the surrounding regions 22 surrounding the colored regions 21, as a whole, form the microparticle-attaching regions 23. In the microparticle-attaching regions 23, the non-water-absorbing, water-insoluble microparticles are attached onto the surfaces of the pulp fibers composing the core wrap 12.

In the microparticle-attaching regions 23, the non-water-absorbing, water-insoluble microparticles attached onto the surfaces of the pulp fibers have an average particle size that is smaller than the average width of the pulp fibers. Since the microparticles have an average particle size that is smaller than the average width of the pulp fibers, there is less of a risk of blocking the gaps between the pulp fibers composing the core wrap 12 and lowering the liquid permeability of the core wrap 12.

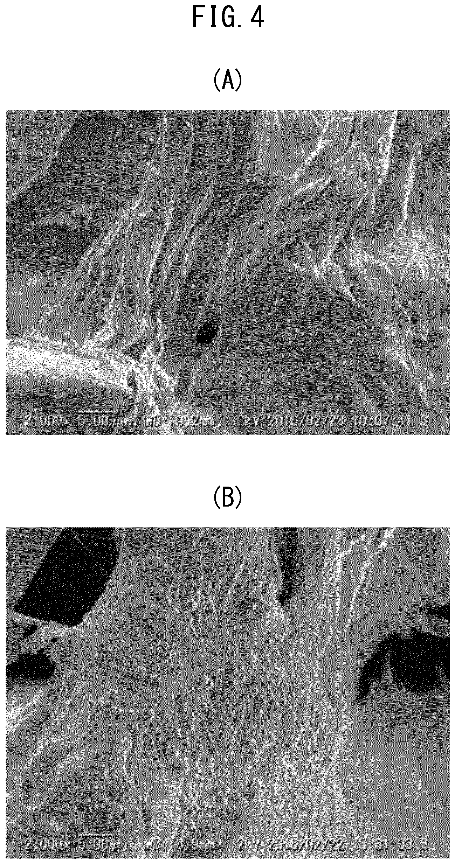

Fig. 4(A) is a photograph of the surface of a core wrap composed of tissue paper containing pulp fibers (Comparative Example 5 below), taken with an electron microscope. Referring to Fig. 4(A), pulp fibers with fiber widths of about 20 µm can be seen, with a gap (space) being apparent between the pulp fibers. Fig. 4(B) is a photograph of the surface of a core wrap coated with a dispersion containing non-water-absorbing, water-insoluble microparticles and a binder on the core wrap shown in Fig. 4(A) (Example 1 below), taken with an electron microscope in the same manner as Fig. 4(A). Referring to Fig. 4(B), a plurality of microparticles are observed to be attaching in a manner covering the surfaces of the pulp fibers. Since the sizes of the microparticles are no greater than about 1 µm, or smaller than the widths of the pulp fibers, it is clear that the microparticles do not block the gaps (spaces) between the fibers.

For this embodiment, the absorbent body 10 has colored regions 21, in the recesses P of the embossed sections, that are visible through the top sheet 13 from the skin side, with the non-water-absorbing, water-insoluble microparticles being coated on the colored regions 21 that are colored blue and their surrounding regions 22, the microparticles being in a state of attaching on the surfaces of the pulp fibers serving as the fiber material. The core wrap is white, but the microparticles are also white and the surrounding regions 22 are white.

In a disposable diaper 1 including such an absorbent body 10, when body fluid such as urine is absorbed into the absorbent body 10 (core wrap 12 and absorbent core 11) through the top sheet 13, the core wrap 12 and absorbent core 11 become stained to the color of the body fluid (yellow, in the case of urine), but with this embodiment, white microparticles are attached onto the surfaces of the pulp fibers of the core wrap 12, and therefore the color of the body fluid is concealed by the white microparticles in both the colored regions 21 and the surrounding regions 22 (the microparticle-attaching regions 23), such that for a person viewing the disposable diaper 1, the degree of contamination of the original color of both the colored regions 21 and the surrounding regions 22 (the microparticle-attaching regions 23) with the color of the body fluid is minimal. Since color contamination (color mixture) is minimal not only in the colored regions 21 but also in the surrounding regions 22, there is less reduction in color contrast between the color of the colored regions 21 and the background color of the surrounding regions 22, and reduction in the visibility of the colored regions 21 can be minimized.

According to this embodiment, referring to Fig. 2 and Fig. 3, the microparticle-attaching regions 23 are the entirety of both the colored regions 21 and the surrounding regions 22 having areas that can encompass the colored regions 21, but it is sufficient if the microparticle-attaching regions, as the background portions of the colored regions, are guaranteed to have areas that can prevent color contamination by body fluid, maintain color contrast with the colored regions and maintain visibility of the colored regions. The areas of the regions surrounding the colored regions will depend on the sizes of the colored regions, but as an example, the areas of the surrounding regions may be at least twice or 4 times or greater, and are preferably 8 times or greater or 10 times or greater, compared to the areas of the colored regions. Moreover, the surrounding regions preferably surround the colored regions at widths of 5 mm or greater, 10 mm or greater, 15 mm or greater or 20 mm or greater, around the entire periphery of the colored regions. However, so long as the areas of the surrounding regions in which the microparticles are attached are sufficiently large, clearly the widths of the regions surrounding the colored regions may be smaller at sections of the periphery of the colored regions. In the case of long-shaped colored regions, similar to the colored regions formed in the recesses P of the embossed sections shown in Fig. 3, the dimensions of the surrounding regions in the lengthwise direction of the long-shaped colored regions need only be essentially longer than the dimensions of the long-shaped colored regions in the lengthwise direction. Moreover, the microparticle-attaching regions do not need to be only limited regions as shown in Fig. 3, and may instead be the entire surface or essentially the entire surface of the core wrap. In particular, as a modified example of this embodiment, a mode may be imagined which has recesses that are lattice-like embossed sections, between the recesses that are the pair of left and right embossed sections acting as curving origins extending in the lengthwise direction, with virtually the entire surface of the absorbent body as a microparticle-attaching region.

The method of forming the colored regions and microparticle-attaching regions in a disposable diaper according to this embodiment may be production that includes a step of forming the microparticle-attaching regions in the core wrap, and a step of forming recesses and using the recesses as colored regions, during embossing of a laminate of the core wrap and the absorbent core using an embossing roll. The disposable diaper of this embodiment may be produced by the same method as for conventional publicly known disposable diapers, except for the step of forming the colored regions and the microparticle-attaching regions.

### (Non-water-absorbing, water-insoluble microparticles)

The non-water-absorbing, water-insoluble microparticles to be used for the invention (hereunder also referred to simply as "microparticles") will now be described.

That the microparticles are "non-water-absorbing" means that when the microparticles are contacted with deionized water, they do not absorb or swell with the water. However, the term "non-water-absorbing" does not strictly imply absolutely no absorption of water, and allows for some trace absorption of water. Most organic resins absorb trace amounts of water and are not actually "water-absorbing". Non-water-absorbing excludes absorbent polymer particles that fill the gaps between the fibers as a result of the microparticles absorbing water and swelling. If the weight increase of the microparticles is no greater than 10% after 1 hour of immersion of the dried microparticles in deionized water, then they are considered "non-water-absorbing", although the weight increase of the microparticles is preferably no greater than 1%. The weight increase of the microparticles is determined by measuring the weight of the microparticles after they have been placed in deionized water and immersed for 10 minutes while gently stirring, and the microparticles that have been removed from the water are subsequently placed on a glass substrate for 30 seconds in an environment of 20°C, 60% RH and allowed to drain, and calculating the difference between this value and the weight of the microparticles before immersion in water.

That the microparticles are water-insoluble means that the microparticles do not dissolve in water, but it does not imply that the microparticles undergo absolutely no dissolution in water and it still allows for some trace dissolution. If the weight decrease of the microparticles is no greater than 5% after 1 hour of immersion of the dried microparticles in water, then they are considered "water-insoluble", although the weight decrease of the microparticles is preferably no greater than 1%. The weight decrease of the microparticles is determined by measuring the weight of the microparticles after they have been placed in deionized water and immersed for 10 minutes while gently stirring, and the microparticles that have been removed from the water are subsequently placed on a glass substrate for 30 seconds in an environment of 20°C, 60% RH and allowed to drain, and calculating the difference between this value and the weight of the microparticles before immersion in water.

The average particle size of the non-water-absorbing, water-insoluble microparticles used is smaller than the average width of the fiber material, and especially the pulp fibers, composing the base sheet. This is in order to cause the microparticles to adhere onto the fiber material and to prevent the microparticles from blocking the gaps between the fibers and reducing the liquid permeability. The average particle size of the microparticles is preferably no greater than 1/2, more preferably no greater than 1/5, and even more preferably no greater than 1/10 or no greater than 1/15 of the average width of the fiber material.

The average particle size of the microparticles need only satisfy the above relationship with the average width of the fiber material, but it is preferably in the range of 0.01 to 5 µm. In order to cause attaching onto the surface of the fiber material composing the base sheet, the average particle size is preferably no greater than 5 µm. The average particle size is preferably small since attaching onto the surface of the fiber material to cover the surface will be facilitated, but an average particle size of smaller than 0.01 µm is not preferred as it will result in difficult handling and increased cost. The average particle size of the microparticles will depend on the width of the fiber material composing the base sheet, but it is in the range of preferably 0.1 to 3 µm and more preferably 0.15 to 1 µm.

The average particle size of the microparticles is measured by an image imaging method. The equivalent circle diameters of at least 10 particles arbitrarily selected from an imaged photograph of the microparticles are measured, and the average value is calculated.

The non-water-absorbing, water-insoluble microparticles used can provide an effect of reducing transmitted light and concealing the color produced by body fluid even if they are semitransparent, but it is preferred if they are essentially opaque. This is in order to produce a greater effect of concealing coloration by body fluid. The non-water-absorbing, water-insoluble microparticles are preferably white or nearly white in color from the viewpoint of the color concealing property, but depending on the color shade of the colored region, even microparticles having a color other than white can produce a concealing effect so long as the color shade of the microparticles can form a background color and contrast with the color shade of the colored region, and therefore there is no limitation to a white system.

The non-water-absorbing, water-insoluble microparticles may be inorganic microparticles or organic microparticles. Inorganic microparticles include calcium carbonate, clay, titanium dioxide, talc, barium sulfate, amorphous silica and alumina. Organic microparticles used may be organic white pigments, such as styrene-based plastic pigments or acrylic plastic pigments, as publicly known synthetic organic pigments, or polyethylene, microcapsules, urea resin or melamine resin. There may also be used phenol resins such as phenolformaldehyde resin, phenolacetaldehyde resin, phenolacetylene resin or terpenephenol resin and their polyvalent metal salts, salicylic acid and its metal salts (especially zinc salt), sulfonylurea compounds, and the like.

The coverage of the non-water-absorbing, water-insoluble microparticles on the base sheet will depend on the density of the fiber material of the base sheet and also on the thickness of the base sheet, but it is preferably 0.35 to 3 g/m² (gsm) and more preferably 0.5 to 2 g/m² (gsm). If the coverage is within this range, it will be possible to cover the fiber material of the base sheet and exhibit a concealing effect without inhibiting the liquid permeability of the base sheet. If the coverage is low, the concealing effect will be reduced. While the liquid permeability of the base sheet will still not be inhibited even if the coverage exceeds this range, the concealing effect will become saturated and the cost will increase.

When the non-water-absorbing, water-insoluble microparticles are attached onto the fiber material of the base sheet, a dispersion of the microparticles may be simply coated onto the base sheet, but it is preferred to use a binder. By binding the microparticles onto the fiber material of the base sheet, the microparticles will be less likely to wash off from the fiber material by body fluid. The binder used may be any of various resins, examples of which include conjugated diene-based latexes, acrylic latexes such as acrylic acid ester and methacrylic acid ester polymer latexes, vinyl-based latexes such as ethylene-vinyl acetate polymer latexes, and synthetic resin-based binders including polyvinyl alcohols, olefin-maleic anhydride resins, melamine resins, urea resins and urethane resins.

### (Base sheet)

According to the invention, the base sheet in which the microparticle-attaching regions are to be formed is a sheet that is liquid-permeable and has a construction including a fiber material. Such a liquid-permeable sheet that is employed is not particularly restricted so long as it is one among those commonly used in the technical field having a construction including a fiber material, and tissue paper, woven fabrics and nonwoven fabrics are examples.

The fibers as the fiber material composing such a woven fabric or nonwoven fabric may be natural fibers or chemical fibers, with examples of natural fibers including cellulose such as ground pulp and cotton, and examples of chemical fibers including regenerated cellulose such as rayon and fibril rayon, semi-synthetic cellulose such as acetate and triacetate, thermoplastic hydrophobic chemical fibers, and hydrophilicized thermoplastic hydrophobic chemical fibers. It most preferably includes pulp fibers.

Tissue paper before absorption of body fluid has a high fiber material density and high coloration density, allowing the colored regions to be easily seen, but after absorption of body fluid the coloration of the colored regions tends to become less visible due to the retained body fluid, and therefore according to the invention, forming the microparticle-attaching regions in a tissue paper base sheet has a greater effect and is preferred.

The (average) width of the fiber material in the base sheet is preferably about 5 to 25 µm. Here, the width of the fiber material is the dimension (width) in the widthwise direction rather than in the lengthwise direction of the fiber material, as viewed in the direction perpendicular to the surface of the base sheet. For a common fiber material, the dimension in the direction of thickness with respect to the lengthwise direction of the fibers of the fiber material will be referred to as the width. For pulp fibers, it is assumed that they have a flat shape, and are flat pulp fibers accumulated in the base sheet, the pulp fiber widths being the short dimensions rather than the long dimensions, as viewed from the direction perpendicular to the flat surfaces of the flat pulp fibers.

The liquid-permeable base sheet may also include an absorbent polymer (SAP) in addition to the fiber material.

The basis weight and composition (proportion) of the fiber material and absorbent polymer (SAP) in the liquid-permeable base sheet, or the density of the base sheet, may be the same as conventionally used in the technical field.

According to the invention, the liquid-permeable base sheet preferably has microparticle-attaching regions formed in a core wrap. For this embodiment, embossed sections coinciding the colored regions are formed in the core wrap, and the resulting effect of rendering the colored regions more easily visible by the microparticle-attaching regions is notable.

### (Other embodiments)

The disposable diaper of the invention may be produced by the same method as for conventional publicly known disposable diapers, except for having the colored regions and microparticle-attaching regions in a specified arrangement.

In the embodiment described above, which was explained with reference to Figs. 1 to 3, the colored regions overlap with the recesses formed in the absorbent core, but it is also preferable that the colored regions overlap with slits formed in the absorbent core.

Also, in the aforementioned embodiment explained with reference to Figs. 1 to 3, the colored regions and microparticle-attaching regions were formed in the core wrap, but it is sufficient if the members forming the colored regions and microparticle-attaching regions are the liquid-permeable base sheet that includes the fiber material, even if they are not in the core wrap.

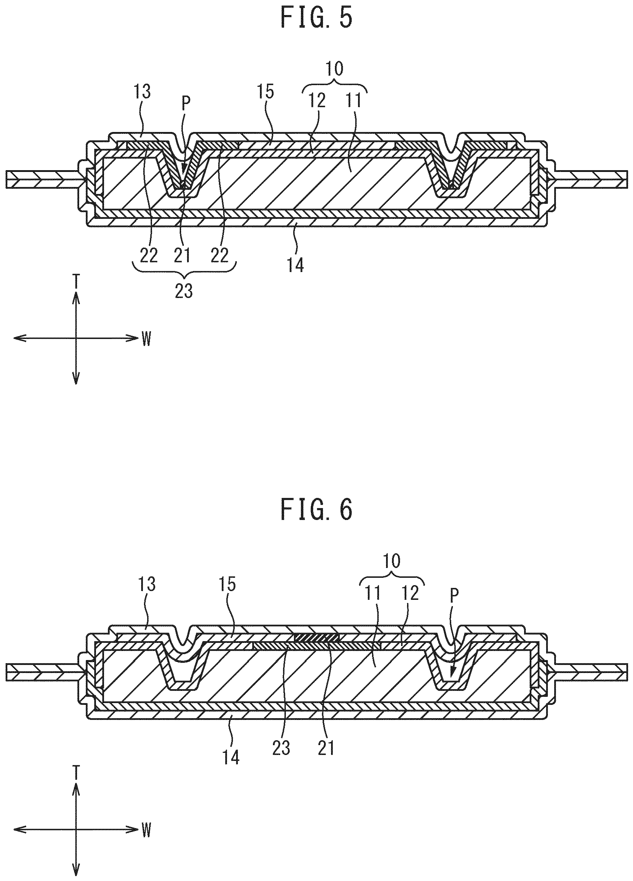

For example, the microparticle-attaching regions and colored regions may be formed in a liquid-permeable second sheet containing the fiber material. This embodiment is shown in Fig. 5. Fig. 5 is an end view of a disposable diaper similar to Fig. 2, where the colored regions 21 and the microparticle-attaching regions 23 (and therefore the surrounding regions 22) are formed not in the core wrap 12 but in the second sheet 15. The recesses P formed by embossing extend from the surface of the second sheet 15 to a depth within the absorbent core 11. The disposable diaper 1 of this embodiment may be produced by a method that includes a step of forming the microparticle-attaching regions in the second sheet, and a step of forming recesses and using the recesses as colored regions, during embossing of a laminate of the absorbent core, core wrap and second sheet using an embossing roll.

Also, unlike the embodiment described above, the microparticle-attaching regions and colored region may be formed in different liquid-permeable sheets. For example, the microparticle-attaching regions may be formed in the core wrap and the colored regions formed in the second sheet. This embodiment is shown in Fig. 6. Fig. 6 is an end view of a disposable diaper similar to Fig. 2, where the colored regions 21 are formed in the second sheet 15 and the microparticle-attaching regions 23 are formed not in the second sheet 15 but in the core wrap 12. Furthermore, although the colored regions 21 and microparticle-attaching regions 23 overlap in the thickness direction T, both are formed in the region between the pair of left and right recesses P formed by embossing, instead of in the recesses P. According to this embodiment, the arrangement of the regions forming the colored regions 21 and the microparticle-attaching regions 23 may be as desired, so long as they mutually overlap in the thickness direction T. The disposable diaper 1 of the embodiment of Fig. 6 may be produced by a method that includes a step of forming the microparticle-attaching regions in the core wrap, a step of embossing a laminate of the absorbent core and core wrap using an embossing roll, a step of forming colored regions in the second sheet, and a step of laminating the laminate of the absorbent core and core wrap (the absorbent body) with the second sheet.

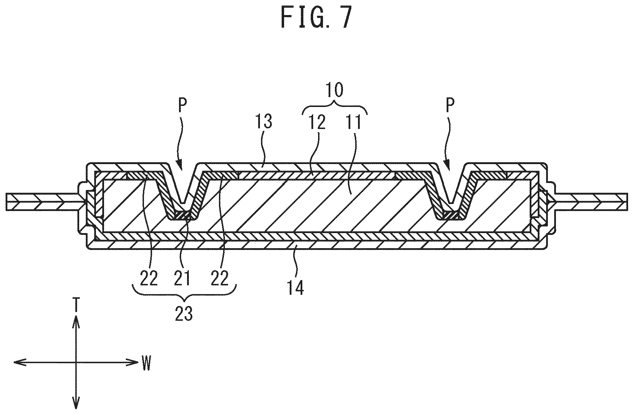

Moreover, in the embodiments illustrated in Fig. 2, Fig. 5 and Fig. 6, the embossing is carried out from above the core wrap or second sheet forming the colored regions, and the second sheet or top sheet is laminated over it, but for any of the modes of the embodiments shown in Fig. 2, Fig. 5 and Fig. 6, the embossing may instead be carried out after lamination of the top sheet. For example, referring to Fig. 7, this differs from the mode shown in Fig. 2 in that there is no second sheet 15, but it also differs in that embossing is carried out from above the top sheet 13 after lamination of the top sheet 13 with the absorbent body 10. However, the colored regions 21 and the surrounding regions 22 and microparticle-attaching regions 23 are formed in the core wrap 12, similar to Fig. 2. It will also be understood that in the embodiments shown in Fig. 5 and Fig. 6 as well, embossing may be carried out from above the top sheet 13 after lamination of the top sheet 13 with the absorbent body 10.

Furthermore, according to a preferred embodiment of the invention, the non-water-absorbing, water-insoluble microparticles used may be non-water-absorbing, water-insoluble microparticles selected from among microparticles known as developing agents (color developing agents). Particularly during formation of recesses by embossing and formation of the colored regions, if a pressure sensitive coloring agent comprising a combination of a color former and a developing agent is used as a coloring agent and the developing agent is coated not only in the colored regions but also in the regions surrounding the colored regions, then during formation of the recesses by embossing and formation of the colored regions in the recesses, it is possible to cause attaching of the non-water-absorbing, water-insoluble microparticles in the colored regions of the recesses and the surrounding regions in the same step, as an embodiment of the invention. If a color former encapsulated in microcapsules is used as a pressure sensitive coloring agent, a developing agent consisting of non-water-absorbing, water-insoluble microparticles is used, and the color former and developing agent are coated at least in the recess-forming regions and the surrounding regions of the base sheet while forming recesses by embossing, then colored regions will be formed in the recesses and the non-water-absorbing, water-insoluble microparticles can be attached onto the surface of the fiber material in the colored regions and the surrounding regions. Coating of the color former onto the base sheet is carried out before embossing, but coating of the developing agent may be carried out either before or after embossing. Also, the colored regions of the base sheet may have the microcapsule color former coated, colored and formed on either the top sheet side surface or the absorbent core side surface of the base sheet.

When the colored regions are to be formed using a pressure sensitive coloring agent during formation of the recesses by embossing on the base sheet and absorbent core, instead of being uniform, coloration at the bottom parts of the recesses may be in a pattern affected by the texture of irregularities and the like in the base sheet. Furthermore, at the slanting wall sections of the recesses, a gradation may be provided wherein the coloration concentration is lower further from the border line between the bottom parts and wall sections of the recesses, by variation in the pressing force. Using a pressure sensitive coloring agent during embossing in this manner is advantageous in that it provides colored regions that coincide with the embossed sections formed in the absorbent body of the absorbent article, while also allowing coloration at the embossed sections to be created in a three-dimensional, natural manner. Moreover, by increasing the pressing force at the locations of the embossing roll that forms the recesses (the plate surface) that are to be the border lines between the bottom parts and wall sections of the recesses that are to be formed (for example, by causing those locations to protrude beyond the other regions on the plate surface) during formation of the colored regions using the pressure sensitive coloring agent, the coloration concentration in the linear regions including the border lines between the bottom parts and the wall sections of the recesses that are to be formed (first linear regions) can be increased above the (average) coloration concentration in linear regions having at least the same widths as the first linear regions on both sides adjacent to the first linear regions (second and third linear regions, respectively). By forming such colored regions, the shapes of the embossed sections can be rendered even more clearly recognizable. Consequently, an absorbent article having colored regions with unique shapes in embossed sections, and non-water-absorbing, water-insoluble microparticle-attaching regions in the colored regions and surrounding regions as described above, can create a definite and three-dimensional or natural impression to help the user perceive the embossed sections, thus providing a hygienic feel and a feeling of assurance.

For the above, the coloration concentration of the colored regions can be evaluated by measuring the L* value in the color chart system (color luminance), from a photographic image of a region including the colored regions. The L* value is 0 for black, and a positive value (usually 100) for white. A high coloration concentration in the colored regions lowers the L* value in the color chart system. The L* value (average value) of the colored regions can be determined by measuring the L* value at 5 arbitrary locations of the embossed sections in the direction perpendicular to the border lines between the bottom parts and the wall sections of the recesses, and calculating the average value. Regarding the coloration concentration, defined as the difference between the L* value for white and the L* value (average value) for the colored regions, the coloration concentration for the first linear regions is preferably at least 15% and more preferably at least 20% higher than the coloration concentration in the color chart system for the second and third linear regions.

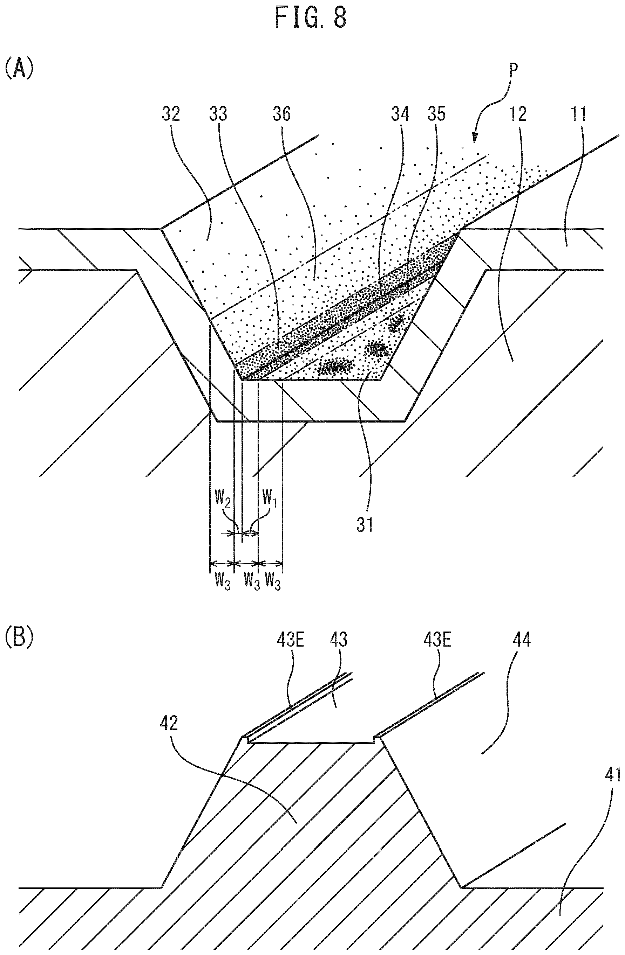

Fig. 8 shows an example of a recess formed by embossing as described above. In Fig. 8(A), the recess P formed in the core wrap 12 and the absorbent core 11 is a groove having a bottom part 31 and wall sections 32. First linear regions 34 with high coloration concentration, represented schematically by dots, lie along the border lines 33 between the bottom part 31 and the wall sections 32, and the coloration concentration in the first linear regions 34 is higher than the coloration concentration in the second linear regions 35 and the third linear regions 36 on either side of it. Each first linear region 34 is an imaginary region delineated so as to have a width w1 on the bottom part 31 side from the border line 33 with the recess P in the planar view, and a width w2 on the wall section 32 side, for a total width w3. The second linear region 35 on the bottom part 31 side adjacent to the first linear region 34 and the third linear region 36 on the wall section 32 side are imaginary regions delineated so as to have width w3, adjacent to the first linear region 34. The first linear region 34, second linear region 35 and third linear region 36 are imaginary regions set with arbitrary widths for measurement of the coloration concentration. In the measurement method described above, if the L* value (average value) for the arbitrary first linear region 34 is lower than the L* value (average value) for the second linear region 35 and third linear region 36, it may be judged to satisfy the condition for the preferred mode described above.

The recess shown in Fig. 8(A) may be formed using an embossing roll having the embossing-forming section shown in Fig. 8(B), and a pressure-sensitive adhesive. In Fig. 8(B), the raised section 42 formed on the base 41 of the embossing roll is the section that forms the recesses P in the embossed sections, and it has a shape that is complementary with the groove-shaped recess P of Fig. 8(A). The raised section 42 has an essentially flat top face 43 that forms the bottom part 31 of the recess P and wall faces 44 that form the wall sections 32 of the recess P, and on the essentially flat top face 43, it has linear regions 43E protruding slightly from the essentially flat top face 43, along the border lines with the wall faces 44. When embossing is carried out using an embossing roll having such raised sections 42 and a pressure-sensitive adhesive, recesses P such as shown in Fig. 8(A) are formed, but since the pressing force at the linear regions 43E protruding from the top face 43 is greater than at the other regions, the coloration concentration can be increased along the border lines between the bottom part and the wall sections of the recesses that are formed, compared to the other regions.

Incidentally, it is evident that the absorbent article of the invention can be applied not only to a disposable diaper but also to other absorbent articles such as incontinence pads or sanitary napkins.

### EXAMPLES

### (Examples 1 to 12 and Comparative Examples 1 to 3)

Ground pulp and an absorbent polymer (SAP) were evenly layered to a basis weight of 270 g/m²(gsm) for the ground pulp and 210 g/m²(gsm) for the absorbent polymer (SAP), and cut to a size of 12 cm × 32 cm. Tissue paper (13 cm × 33 cm), 16 g/m²(gsm), was attached to the top and bottom of the laminate using a hot-melt adhesive, and the thickness was adjusted to 3.0 mm with a pressing machine to fabricate an absorbent body sample.

A dispersion of zinc 3,5-di(α-methylbenzyl)salicylate (BR-054 by Sankosha) was used as a coating solution of non-absorbent, water-insoluble microparticles, and its composition was as follows.

| | |
|---|---|
| Zinc3,5-di(α-methylbenzyl)salicylate | 35 wt% |
| 10-[(Styrene)-(α-methylstyrene)] copolymer 9,10-dihydro-9-oxa-10-phosphaphenanthrene | 7 wt% |
| Polyvinyl alcohol | 1wt% |
| Water | 57 wt% |

The dispersion was used as the stock solution and water was used for dilution to prepare a coating solution of non-absorbent, water-insoluble microparticles at a solid concentration of 35 wt% to 3 wt%.

The coating solution at different concentrations was spray-coated over the entire surface of the absorbent body sample (area: 429 cm²) using a coating applicator (Easy Painter by Gaianotes, Inc.) at a coating amount within the prescribed range, to fabricate absorbent body samples having different microparticle coating amounts (design basis weights). The amount of actual weight increase of the absorbent body during this time was measured and the coating basis weight of the microparticles (measured) was calculated from the solid concentration of the coating solution used.

For Comparative Examples 1 to 3, three absorbent body samples were prepared having water alone coated in the same coating amount as the coating solution of the Example.

The coating basis weights (measured) of the microparticles of each of the examples and comparative examples are shown in Table 1.

### (Absorption rate and concealing property test)

Artificial urine was poured onto and absorbed into the absorbent body samples coated with microparticles (Examples) and the absorbent body samples coated with water alone without microparticles (Comparative Examples) as described above, and the absorption rates and the colors of the absorbent body samples before and after absorption were measured.

The artificial urine was prepared as colored artificial urine, by dissolving 200 g of urea, 80 g of sodium chloride (salt), 8 g of magnesium sulfate·heptahydrate, 3 g of calcium chloride·dihydrate and 1 g of a pigment: Blue #1 component [65% Blue #1/35% sodium chloride] in 10 L of ion-exchanged water.

Using the absorbent body sample and the artificial urine, a cylinder with a 5 cm inner diameter was placed at the center of the microparticle-coated absorbent body, 40 mL of artificial urine was poured in with a burette, and the time from initial pouring in of the artificial urine until the artificial urine in the cylinder disappeared was measured to evaluate the absorption rate.

The cylinder was then removed, and at 3 minutes and 60 minutes thereafter, a colorimeter (CR-300: by Minolta) was used to measure the color (ΔE) on the surface of the absorbent body sample, evaluating the concealing property based on the color difference. The average of measurements taken at 5 locations for each sample at a section of the inner side of the cylinder was recorded as the measured value. ΔE is calculated as ΔE = {(L*)² + (a*)² + (b*)²}^{1/2}, according to L^{∗}a^{∗}b^{∗} in the color chart system.

The test results are shown in Table 1. The ΔE values for the absorbent body samples before the moisture absorption test are also shown in Table 1.

### [Table 1]

**Table 1 Relationship between microparticle coating amount, absorption rate and ΔE**

| | Microparticle-coating amount (gsm) | Absorption rate (sec) | ΔE (before absorption of artificial urine) | ΔE (5 min. later) | ΔE (60 min. later) |
|---|---|---|---|---|---|
| Example 1 | 3.10 | 18.3 | 1.96 | 39.89 | 33.88 |
| Example 2 | 3.10 | 16.8 | 2.11 | 44.39 | 36.41 |
| Example 3 | 1.96 | 16.7 | 2.22 | 41.75 | 33.70 |
| Example 4 | 1.19 | 16.2 | 2.07 | 42.99 | 36.88 |
| Example 5 | 1.33 | 18.5 | 2.10 | 42.18 | 33.00 |
| Example 6 | 1.05 | 15.7 | 2.17 | 44.47 | 37.01 |
| Example 7 | 1.19 | 17.2 | 2.15 | 38.85 | 32.52 |
| Example 8 | 1.21 | 15.5 | 2.02 | 45.15 | 35.66 |
| Example 9 | 0.49 | 17.6 | 2.37 | 49.93 | 41.17 |
| Example 10 | 0.52 | 15.0 | 2.24 | 47.45 | 38.59 |
| Example 11 | 0.49 | 17.9 | 2.37 | 48.36 | 39.39 |
| Example 12 | 0.36 | 15.2 | 2.15 | 52.75 | 41.71 |
| Comp. Ex. 1 | 0.00 | 18.0 | 2.34 | 54.28 | 51.49 |
| Comp. Ex. 2 | 0.00 | 15.5 | 2.41 | 54.86 | 52.94 |
| Comp. Ex. 3 | 0.00 | 16.5 | 2.40 | 56.10 | 52.26 |

According to Table 1, even when non-absorbent, water-insoluble microparticles were coated onto each absorbent body of the examples, there was virtually no reduction in the absorption rate of the absorbent body compared to the comparative examples and no adverse effect on absorption performance of the absorbent body was observed. The color of the absorbent body had increased ΔE after absorption of colored artificial urine, but a significant effect of concealing the color of colored artificial urine was observed in the examples compared to the comparative examples.

The core wrap of Example 1 coated with non-absorbent, water-insoluble microparticles was also observed with an electron microscope. It was found that since the particle sizes of the microparticles (average: 0.6 µm) were sufficiently smaller than the widths of the pulp fibers of the core wrap (average: 20 µm), the microparticles attached to the surfaces of the pulp fibers and did not block the gaps between the pulp fibers. Reference may be made to Fig. 4, for example, which shows electron micrographs of the surfaces of absorbent bodies of Example 1 and a comparative example.

### REFERENCE SIGNS LIST

1 Disposable diaper
10 Absorbent body
11 Absorbent core
12 Core wrap
13 Top sheet
14 Back sheet
15 Second sheet
P Recess
21 Colored region
22 Surrounding region
23 Microparticle-attaching region

## Claims

1. An absorbent article having a liquid-permeable top sheet, a liquid-impermeable back sheet and an absorbent core situated between them, and having a thickness direction, wherein the absorbent article:
has, between the top sheet and the absorbent core, a colored region that is visible through the top sheet and has a different color than the color of the top sheet,
has, between the top sheet and the absorbent core, a liquid-permeable base sheet, the base sheet being constructed to include a fiber material, and
has a microparticle-attaching region in which non-absorbent, water-insoluble microparticles having a different color than the color of the colored region have been attached to the fiber material of the base sheet, the average particle size of the microparticles being smaller than the average width of the fiber material of the base sheet, the coverage of the microparticles being 0.35 to 3 g/m², and
the microparticle-attaching region includes a region that overlaps with both the colored region and the surrounding region surrounding the colored region, in the thickness direction.

2. The absorbent article according to claim 1, wherein the average particle size of the microparticles is in the range of 0.01 to 5 µm.

3. The absorbent article according to claim 1 or 2, wherein the colored region overlaps with recesses and/or slits formed in the absorbent core, in the thickness direction of the absorbent article.

4. The absorbent article according to any one of claims 1 to 3, wherein the microparticles are attached to the fiber material of the base sheet by a binder.

5. The absorbent article according to any one of claims 1 to 4, wherein the colored region is in the base sheet.

6. The absorbent article according to any one of claims 1 to 5, which further has a separate liquid-permeable base sheet different from the base sheet, further toward the top sheet side than the base sheet between the top sheet and the absorbent core, the colored region being in the separate base sheet.

7. The absorbent article according to claim 5, wherein the colored region is colored by a pressure sensitive coloring agent, and the microparticles are a developing agent for the pressure sensitive coloring agent.

8. The absorbent article according to any one of claims 1 to 7, wherein the base sheet is tissue paper.

9. The absorbent article according to any one of claims 1 to 8, wherein the absorbent core is covered by a core wrap, the base sheet is the core wrap, and the colored region is in an embossed section formed in the core wrap and the absorbent core.

## Patentansprüche

1. Absorbierender Artikel, der eine flüssigkeitsdurchlässige obere Lage, eine flüssigkeitsundurchlässige hintere Lage und einen Saugkern, der sich zwischen diesen befindet, aufweist und eine Dickenrichtung aufweist, wobei der absorbierende Artikel:
zwischen der oberen Lage und dem Saugkern einen farbigen Bereich aufweist, der durch die obere Lage sichtbar ist, und eine andere Farbe aufweist als die Farbe der oberen Lage,
zwischen der oberen Lage und dem Saugkern eine flüssigkeitsdurchlässige Grundlage aufweist, wobei die Grundlage aufgebaut ist, dass sie ein Fasermaterial einschließt, und
einen Mikropartikelanbringungsbereich aufweist, in dem nichtabsorbierende, wasserunlösliche Mikropartikel, die eine andere Farbe aufweisen als die Farbe des farbigen Bereichs, an das Fasermaterial der Grundlage angebracht wurden, wobei die durchschnittliche Partikelgröße der Mikropartikel kleiner ist als die durchschnittliche Breite des Fasermaterials der Grundlage, wobei die Bedeckung der Mikropartikel 0,35 bis 3 g/m² beträgt, und
der Mikropartikelanbringungsbereich einen Bereich einschließt, der mit sowohl dem farbigen Bereich als auch dem umgebenden Bereich, der den farbigen Bereich umgibt, in der Dickenrichtung überlappt.

2. Absorbierender Artikel nach Anspruch 1, wobei die durchschnittliche Partikelgröße der Mikropartikel in dem Bereich von 0,01 bis 5 µm liegt.

3. Absorbierender Artikel nach Anspruch 1 oder 2, wobei der farbige Bereich mit Vertiefungen und/oder Schlitzen, die in dem Saugkörper ausgebildet sind, in der Dickenrichtung des absorbierenden Artikels überlappt.

4. Absorbierender Artikel nach einem der Ansprüche 1 bis 3, wobei die Mikropartikel an dem Fasermaterial der Grundlage durch ein Bindemittel angebracht sind.

5. Absorbierender Artikel nach einem der Ansprüche 1 bis 4, wobei der farbige Bereich in der Grundlage vorliegt.

6. Absorbierender Artikel nach einem der Ansprüche 1 bis 5, der weiter eine separate flüssigkeitsdurchlässige Grundlage, die sich von der Grundlage unterscheidet, weiter in Richtung der oberen Lagenseite als die Grundlage zwischen der oberen Lage und dem Saugkörper aufweist, wobei der farbige Bereich in der separaten Grundlage vorliegt.

7. Absorbierender Artikel nach Anspruch 5, wobei der farbige Bereich durch einen druckempfindlichen Farbstoff gefärbt ist und die Mikropartikel ein Entwicklermittel für den druckempfindlichen Farbstoff sind.

8. Absorbierender Artikel nach einem der Ansprüche 1 bis 7, wobei die Grundlage Tissue-Papier ist.

9. Absorbierender Artikel nach einem der Ansprüche 1 bis 8, wobei der Saugkern von einer Kernumhüllung bedeckt ist, die Grundlage die Kernumhüllung ist und der farbige Bereich in einem geprägten Abschnitt vorliegt, der in der Kernumhüllung und dem Saugkern ausgebildet ist.

## Revendications

1. Article absorbant ayant une feuille de dessus perméable aux liquides, une feuille de support imperméable aux liquides et une partie centrale absorbante située entre elles, et ayant une direction allant dans le sens de l'épaisseur, dans lequel l'article absorbant :
a, entre la feuille de dessus et la partie centrale absorbante, une région colorée qui est visible au travers de la feuille de dessus et qui a une couleur différente par rapport à la couleur de la feuille de dessus,
a, entre la feuille de dessus et la partie centrale absorbante, une feuille de base perméable aux liquides, la feuille de base étant construite pour comprendre un matériau fibreux, et
a une région d'attache de microparticules dans laquelle des microparticules non absorbantes et insolubles dans l'eau ayant une couleur différente par rapport à la couleur de la région colorée ont été attachées au matériau fibreux de la feuille de base, la dimension moyenne des particules des microparticules étant inférieure à la largeur moyenne du matériau fibreux de la feuille de base, le pouvoir couvrant des microparticules allant de 0,35 à 3 g/m², et
la région d'attache de microparticules comprend une région qui chevauche à la fois par rapport à la région colorée et à la région environnante qui entoure la région colorée, dans la direction allant dans le sens de l'épaisseur.

2. Article absorbant selon la revendication 1, dans lequel la dimension moyenne des particules des microparticules se trouve dans la plage allant de 0,01 à 5 µm.

3. Article absorbant selon la revendication 1 ou la revendication 2, dans lequel la région colorée chevauche par rapport à des évidements et/ou des fentes formés dans la partie centrale absorbante, dans la direction allant dans le sens de l'épaisseur de l'article absorbant.

4. Article absorbant selon l'une quelconque des revendications 1 à 3, dans lequel les microparticules sont attachées au matériau fibreux de la feuille de base par un liant.

5. Article absorbant selon l'une quelconque des revendications 1 à 4, dans lequel la région colorée est dans la feuille de base.

6. Article absorbant selon l'une quelconque des revendications 1 à 5, qui a par ailleurs une feuille de base séparée perméable aux liquides qui est différente de la feuille de base, se trouvant plus loin vers le côté de la feuille de dessus par rapport à la feuille de base entre la feuille de dessus et la partie centrale absorbante, la région colorée étant dans la feuille de base séparée.

7. Article absorbant selon la revendication 5, dans lequel la région colorée est colorée par un agent colorant sensible à la pression, et les microparticules sont un agent développant pour l'agent colorant sensible à la pression.

8. Article absorbant selon l'une quelconque des revendications 1 à 7, dans lequel la feuille de base est du papier de soie.

9. Article absorbant selon l'une quelconque des revendications 1 à 8, dans lequel la partie centrale absorbante est recouverte au moyen d'une enveloppe pour partie centrale, la feuille de base est l'enveloppe pour partie centrale, et la région colorée est dans une section gaufrée formée dans l'enveloppe pour partie centrale et la partie centrale absorbante.
